# EUROPEAN PATENT SPECIFICATION

(11) **EP 3 287 129 B1**
(45) Date of publication and mention of the grant of the patent: **06.08.2025**
(21) Application number: 16782980.3
(22) Date of filing: 04.04.2016
(51) Int. Cl.: A61K 31/19, A61K 31/191, A61K 31/201, A61K 39/39, A61P 37/04, A61P 43/00

(54) **3-HYDROXYDECANOIC ACID AND/OR HYDROXYDECANOIC ACID FOR USE IN MUCOSAL IMMUNOSTIMULATION**
3-HYDROXYDECANSÄURE UND/ODER HYDROXYDECANOIDSÄURE ACID ZUR VERWENDUNG ZUR MUKOSALEN IMMUNOSTIMULATION
L'ACIDE 3-HYDROXYDECANOIQUE ET/OU L'ACIDE HYDROXYDECANOIQUE ET SON UTILISATION DANS LA STIMULATION IMMUNITAIRE MUCOSALE

(30) Priority: 22.04.2015 JP 2015087644
(43) Date of publication of application: 28.02.2018
(73) Proprietor: Yamada Bee Company, Inc., Tomata-gun, Okayama 708-0393 (JP); National University Corporation Kumamoto University, Kumamoto-shi, Kumamoto 860-8555 (JP)
(72) Inventor: MISUMI, Shogo, Kumamoto-shi Kumamoto 862-0973 (JP); IKUTA, Tomoki, Tomata-gun Okayama 708-0393 (JP); TATEFUJI, Tomoki, Tomata-gun Okayama 708-0393 (JP)
(74) Representative: Grünecker Patent- und Rechtsanwälte PartG mbB
(86) International application number: PCT/JP2016/061022
(87) International publication number: WO 2016/170959

(56) References cited:
- WO-A1-00/51634
- JP-A- 2009 274 961
- JP-A- H0 952 816
- TAKAHASHI K ET AL: "Pharmacokinetic analysis of the absorption enhancing action of decanoic acid and its derivatives in rats.", PHARMACEUTICAL RESEARCH MAR 1994, vol. 11, no. 3, March 1994 (1994-03-01), pages 388 - 392, XP002786863, ISSN: 0724-8741
- SVER L ET AL: "A royal jelly as a new potential immunomodulator in rats and mice.", COMPARATIVE IMMUNOLOGY, MICROBIOLOGY AND INFECTIOUS DISEASES JAN 1996, vol. 19, no. 1, January 1996 (1996-01-01), pages 31 - 38, XP002786864, ISSN: 0147-9571
- GASIC SONJA ET AL.: "Evaluation of the immunomodelatory Activities of Royal Jelly Components In Vitro", IMMUNOPHARMACOLOGY AND IMMUNOTOXICOLOGY, vol. 29, no. 3-4, 2007, pages 521 - 536, XP009506887
- MIHAJLOVIC DUSAN ET AL.: "Royal jelly fatty acids modulate proliferation and cytokine production by human peripheral blood mononuclear cells", EUR. FOOD RES. TECHNOL., vol. 238, no. 5, 2014, pages 881 - 887, XP035317191
- CHEN, CHINA -JUNG ET AL.: "Structure and functions of gamma-dodecalactone isolated from Antrodia camphorata for NK cell activation", BIOORGANIC & MEDICINAL CHEMISTRY, vol. 18, no. 18, 2010, pages 6896 - 6904, XP027252791

## Description

### Technical Field

The present invention relates to a novel mucosal immunostimulator.

### Background Art

Mucous tissues such as the intestinal tract or respiratory organs, which is in contact with the external environment, become initial infection sites in many cases. Therefore, it is important, from the viewpoint of preventive medicine, to strengthen the mucosal immune function. As the front barrier of infection prevention mechanism, Peyer patches and nasal-associated lymphoid tissue (NALT) play central roles in the induction and control of mucosal immune responses. Microfold cells (M cells) are found in gut-associated lymphoid tissue, such as Peyer patches, and the follicle-associated epithelium (FAE) covering lymphoid follicles, such as NALT. M cells are involved in the initiation of mucosal immune responses through the uptake and transcytosis of intestinal antigens (Non Patent Literature 1). Glycoprotein 2 (GP2) is known as an M cell-specific marker (Non Patent Literature 2).

As a mucosal immunostimulator, Patent Literature 1 discloses a mucosal immunostimulator comprising, as an active ingredient, at least one honeybee product selected from royal jelly, propolis, and bee pollen.

### Citation List

### Patent Literature

Patent Literature 1: JP 2011-84525 A

### Non Patent Literature

Non Patent Literature 1: Bioscience of Microbiota, Food and Health, 2014, Vol. 33 (No. 3), pp. 91-97
Non Patent Literature 2: Mucosal Immunology, 2013, Vol. 6 (No. 4), pp. 666-677
JP-A-2009-274961 relates to absorption promoters for enhancing absorption efficiency of medicine through a mucous membrane to an organism.
K. Takahashi et al., Pharmaceutical Research 1994, 11, 388 to 392 describes the absorption enhancing action of decanoic acids.
Sver et al., Comp. Immun. Microbiol. infect. Dis 1996, 19, 31 to 38 studied possible immunomodulatory effects of royal jelly.

### Summary of Invention

### Technical Problem

Some mucosal immunostimulators are known, as disclosed in Patent Literature 1; however, in order to satisfy various consumers' needs, it cannot be said that there have been sufficient options. Accordingly, an object of the present invention is to provide a novel mucosal immunostimulator.

### Solution to Problem

The present invention relates to an agent comprising, as an active ingredient, at least one selected from the group consisting of 3-hydroxydecanoic acid and 10-hydroxydecanoic acid and salts thereof for use in mucosal immunostimulation.

Because the mucosal immunostimulator of the present invention comprises, as an active ingredient, at least one selected from 3-hydroxydecanoic acid and 10-hydroxydecanoic acid and salts thereof, it can stimulate mucosal immunity.

It is even more preferable that the saturated fatty acid be 10-hydroxydecanoic acid. 10-Hydroxydecanoic acid is also known as a constituent of royal jelly, has high safety for the organism, and can be continuously taken for a long period of time.

Because the mucosal immunostimulator of the present invention comprises, as an active ingredient, at least one selected from 3-hydroxydecanoic acid and 10-hydroxydecanoic acid and salts thereof, it can induce the differentiation of M cells. At least part of the action mechanism by the mucosal immunostimulator of the present invention is based on the induction of differentiation of M cells.

The present invention further relates to the above mucosal immunostimulator as an adjuvant. Because the above mucosal immunostimulator can induce the differentiation of M cells involved in the initiation of mucosal immune responses, it can be suitably used as an adjuvant.

The present invention further relates to a vaccine preparation comprising an immunogen and at least one agent selected from 3-hydroxydecanoic acid and 10-hydroxydecanoic acid for use as a vaccine, whereby the use includes mucosal immunostimulation.

The present invention can also be regarded as 3-hydroxydecanoic acid and 10-hydroxydecanoic acid or salts thereof for use in mucosal immunostimulation. The present invention can also be regarded as an agent comprising, as an active ingredient, at least one selected from 3-hydroxydecanoic acid and 10-hydroxydecanoic acid and salts thereof for use in mucosal immunostimulation.

### Advantageous Effects of Invention

A novel mucosal immunostimulator is provided by the present invention. The mucosal immunostimulator of the present invention can induce the differentiation of M cells, and is thus useful as an adjuvant.

### Brief Description of Drawings

Fig. 1 is histograms of FACS showing the results of an M-cell differentiation induction test.
Fig. 2 is an electron micrograph showing the results of an M-cell differentiation induction test.
Fig. 3 is fluorescent immunohistostaining photographs showing the results of an M-cell differentiation induction test.
Fig. 4 is fluorescent immunohistostaining photographs showing the results of an immunostimulation test.
Fig. 5 is graphs showing the measurement results of antibody titers.

### Description of Embodiments

Hereinafter, embodiments for carrying out the present invention are described in detail.

The mucosal immunostimulator of the present invention comprises, as an active ingredient, at least one selected from the group consisting of 3-hydroxydecanoic acid and 10-hydroxydecanoic acid

When an optical isomer is present, the saturated fatty acids may be either of R-form and S-form.

The 3-hydroxydecanoic acid and 10-hydroxydecanoic acid may form salts with alkali metals, alkaline earth metals, other metals, or ammonium, that are acceptable for food applications or pharmaceutical applications. Specific examples include potassium salts, sodium salts, calcium salts, and magnesium salts.

The above 3-hydroxydecanoic acid and 10-hydroxydecanoic acid and salts thereof can be produced according to a conventional method, such as a method involving alkali fusion of recinoleic acid contained in castor oil (e.g., Eur. J. Lipid Sci. Technol., 2010, 112(1), pp. 10-30; or J. Am. Oil Chem. Soc., 1974, 51(3), pp. 65-71). Alternatively, commercially available products may also be used.

The mucosal immunostimulator of the present invention may comprise one of the above 3-hydroxydecanoic acid and 10-hydroxydecanoic acid and salts thereof singly or two or more in combination.

The mucosal immunostimulator of the present invention may comprise only the above active ingredient, or may further comprise other ingredients as long as the effects of the present invention are not impaired. Examples of other ingredients include pharmaceutically acceptable ingredients (e.g., diluents, binders, lubricants, disintegrators, emulsifiers, surfactants, bases, solubilizing agents, and suspending agents) and ingredients acceptable as food (e.g., minerals, vitamins, flavonoids, quinones, polyphenols, amino acids, nucleic acids, essential fatty acids, fresheners, binders, sweeteners, disintegrators, lubricants, coloring agents, flavoring agents, stabilizers, preservatives, sustained release controlling agents, surfactants, solubilizing agents, and wetting agents).

The mucosal immunostimulator of the present invention can be used, in terms of the amount of active ingredient, at a dose of 1 mg or more and 10 g or less per day for an adult with a body weight of 60 kg, preferably a dose of 5 mg or more and 8 g or less, more preferably a dose of 10 mg or more and 3 g or less, even more preferably a dose of 15 mg or more and 1.5 g or less, further more preferably a dose of 20 mg or more and 1 g or less, further even more preferably a dose of 22 mg or more and 500 mg or less, and particularly preferably a dose of 24 mg or more and 250 mg or less. The dose of the mucosal immunostimulator of the present invention when used in an infant may be, for example, 3/5 of the adult dose for infants at an age of 6 to less than 13 years old, 2/5 of the adult dose for infants at an age of 1 to less than 6 years old, and 1/5 of the adult dose for infants at an age of less than 1 year old. This dose can be suitably determined within the above-mentioned range, depending on the factors such as health conditions of a person to take, administration route, combination with other agents.

The mucosal immunostimulator of the present invention may be orally administered (taken), or intranasally administered. The mucosal immunostimulator of the present invention may be administered once a day, or administered in several times a day, such as twice a day or three times a day, as long as the amount of active ingredient per day is within the above range. It is preferable that the mucosal immunostimulator of the present invention be continuously administered. Mucosal immunostimulating effects are more significantly exhibited by continuous administration.

The mucosal immunostimulator of the present invention may be in any form, such as a solid, liquid, or paste. Examples of the form of the mucosal immunostimulator of the present invention include uncoated pills, sugar-coated pills, granules, powders, tablets, and capsules (hard capsules, soft capsules, and seamless capsules). The mucosal immunostimulator of the present invention can be prepared by, for example, mixing an active ingredient, which is a saturated fatty acid or a salt thereof, with other ingredients, if necessary, and forming the mixture into a dosage form mentioned above.

The mucosal immunostimulator of the present invention can be used as it is as a drug, a quasi-drug, or a food composition, and also can be used to be added to drugs, quasi-drugs, and food compositions. It is preferable that the food composition be food in which the third function (physical condition-controlling function) of food is strengthened. Examples of the food in which the third function of food is strengthened include health foods, foods with function claims, nutritional supplementary foods, supplements, and foods for specified health uses.

A drug, a quasi-drug, or a food composition, each of which consists of the mucosal immunostimulator of the present invention, or a drug, a quasi-drug, or a food composition, each of which comprises the mucosal immunostimulator of the present invention, may be used for mucosal immunostimulation, and may be labelled with the following indications: "increasing immune activity," "making the body resistant to viruses," "supporting immune function," "being useful to increase M cells," "keeping immune activity," "maintaining immune activity," "being suitable for people who easily catch cold," "making immune function excellent," "being useful to prevent weakened immune systems from being further weakened," "making the body resistant to food-poisoning bacteria," "supporting the health of the mucosa," "supporting mucosal immunity," "supporting the health of the nose and throat," "being useful for maintenance of health during winter," "being useful for maintenance of health in cold seasons," and "making the body withstanding cold seasons,".

The content of the mucosal immunostimulator of the present invention in drugs, quasi-drugs, and food compositions may be suitably determined depending on the type of drug, quasi-drug, and food composition, so that the amount of active ingredient taken per day is within the above range.

When the mucosal immunostimulator of the present invention is used as it is as a food composition, or used to be added to a food composition, the form of the food composition include beverages (soft drinks such as coffee, juice, and tea, milk drinks, lactic acid bacteria drinks, yogurt drinks, carbonated drinks); spreads (custard cream); pastes (fruit paste); Western confectionary (chocolates, doughnuts, pies, cream puffs, gums, jellies, candies, cookies, cakes, puddings); Japanese confectionary (daifuku, mochi, manju, castella, anmitsu, yokan); frozen desserts (ice cream, popsicle, sherbet); food products (curry, beef bowl, porridge, miso soup, soup, meat sauce, pasta, pickles, jams); and seasonings (dressing, furikake, tasty seasoning, soup stock).

When the mucosal immunostimulator of the present invention is used as it is as food in which the third function of food is strengthened (e.g., health foods, foods with function claims, nutritional supplementary foods, supplements, or foods for specified health uses), or used to be added to food in which the third function of food is strengthened, examples of the form of the food in which the third function of food is strengthened include, in addition to the above forms of food, uncoated pills, sugar-coated pills, granules, powders, tablets, and capsules (hard capsules, soft capsules, and seamless capsules).

When the mucosal immunostimulator of the present invention is used as it is as a drug or a quasi-drug, or when the mucosal immunostimulator of the present invention is used to be added to a drug or a quasi-drug, the form of the drug or quasi-drug is not particularly limited, and examples thereof include uncoated pills, sugar-coated pills, granules, powders, tablets, and capsules (hard capsules, soft capsules, and seamless capsules).

The method for producing drugs, quasi-drugs, or food compositions, to each of which the mucosal immunostimulator of the present invention is added, can be suitably performed according to a known method. For example, drugs, quasi-drugs, or food compositions used for the above applications can be obtained by, for example, mixing the mucosal immunostimulator of the present invention with intermediate products or final products in the production process of the drugs, quasi-drugs, or food compositions.

The present invention can also be regarded as 3-hydroxydecanoic acid and 10-hydroxydecanoic acid or salts thereof for use in mucosal immunostimulation. The present invention can also be regarded as an agent comprising, as an active ingredient, at least one selected from the group consisting of 3-hydroxydecanoic acid and 10-hydroxydecanoic acid or salts thereof for use in mucosal immunostimulation.

### [Vaccine Preparation]

The mucosal immunostimulator of the present invention can also be used as an adjuvant. At least part of the action mechanism by the adjuvant of the present invention is based on the induction of differentiation of M cells, which is involved in the initiation of mucosal immune responses. The adjuvant according to the present embodiment may be one comprising the above mucosal immunostimulator or one consisting of the above mucosal immunostimulator.

The vaccine preparation of the present invention comprises the above agent for use in mucosal immunostimulation as an adjuvant. The adjuvant of the present invention can be combined with various immunogens to form vaccine preparations. The vaccine preparation of the present invention may be a mixture of the adjuvant and an immunogen, or the adjuvant and an immunogen may be separated so that they are mixed at the time of administration or separately administered. The vaccine preparation of the present invention may be used for treatment or for prevention.

The immunogen to be used in combination with the adjuvant of the present invention is not particularly limited, as long as it is an antigen that induces an immune response against mammals, such as humans. Specific examples of immunogens include one or more pathogenic microorganisms selected from the group consisting of influenza virus, rotavirus, adenovirus, norovirus, measles virus, rubella virus, mumps virus, AIDS virus, hepatitis virus, poliovirus, rabies virus, Bordetella pertussis, Corynebacterium diphtheriae, Helicobacter pylori, enterohaemorrhagic Escherichia coli (EHEC), Chlamydia protozoa, Mycoplasma protozoa, Malaria parasite, coccidium protozoa, and schistosome; antigens derived from these pathogenic microorganisms, and prion proteins. These immunogens may be inactivated or not inactivated.

The quantitative ratio of immunogen and adjuvant in the vaccine preparation of the present invention can be suitably determined depending on the type of immunogen used, the purpose of vaccine preparation, etc. For example, the quantitative ratio may be 1:0.0001 to 1:10000 (weight ratio), and is preferably 1:0. 1 to 1:10 (weight ratio).

The form of the vaccine preparation of the present invention may be liquid or powder. The form of the vaccine preparation of the present invention is not particularly limited, and examples thereof include uncoated pills, sugar-coated pills, granules, powders, tablets, and capsules (hard capsules, soft capsules, and seamless capsules).

Known stabilizers, preservatives, etc., may be contained in the vaccine preparation of the present invention. The vaccine preparation of the present invention can be produced by a known method. The vaccine preparation of the present invention may be administered orally or intranasally.

The vaccine preparation of the present invention can be administered by a method comprising a step of administering the adjuvant to a subject, and a step of administering an immunogen to the subject. Administration of the adjuvant and administration of an immunogen may be performed simultaneously or separately. For example, administration of the adjuvant can be previously performed, and administration of an immunogen can be then performed. Furthermore, administration of an immunogen can be performed in a timely manner while the adjuvant is continuously administered. It is preferable that the vaccine preparation of the present invention be used such that, for example, an immunogen is administered after the adjuvant is continuously administered. Induction of immune responses can thereby be further facilitated. As a specific example of an embodiment in which the adjuvant is continuously administered, administration of an effective amount per day is continued for 3 days or more, preferably 7 days or more, more preferably 14 days or more, and even more preferably 21 days or more. Administration may be conducted in several times a day, such as twice a day or three times a day, within the effective amount range per day.

The effective amount of the adjuvant is, in terms of the amount of active ingredient, a dose of 1 mg or more and 10 g or less per day for an adult with a body weight of 60 kg, preferably a dose of 5 mg or more and 8 g or less, more preferably a dose of 10 mg or more and 3 g or less, even more preferably a dose of 15 mg or more and 1.5 g or less, further more preferably a dose of 20 mg or more and 1 g or less, further even more preferably a dose of 22 mg or more and 500 mg or less, and particularly preferably a dose of 24 mg or more and 250 mg or less. The effective amount for an infant may be, for example, 3/5 of the adult dose for infants at an age of 6 to less than 13 years old, 2/5 of the adult dose for infants at an age of 1 to less than 6 years old, and 1/5 of the adult dose for infants at an age of less than 1 year old. This dose can be suitably determined within the above-mentioned range, depending on the factors such as health conditions of a person to take, administration route, combination with other agents.

### Examples

Hereinafter, the present invention is described in more detail based on Examples.

### [Test Example 1: M-Cell Differentiation Induction Test - in vitro Test]

### [Cell Culture]

As an in vitro M-cell model, Caco-2 cells derived from human intestinal epithelium cells were used. The Caco-2 cells were cultured in an EMEM medium (produced by Nissui Pharmaceutical Co., Ltd.) supplemented with 2% fetal calf serum (FCS, produced by CANSERA INTERNATIONAL), 2 mM glutamine (produced by Wako Pure Chemical Industries, Ltd.), and 0.1 mM nonessential amino acid (produced by Gibco) at 37°C in the presence of 5% CO₂. The cells were subcultured within a range of logarithmic growth (2.0 x 10⁵ to 1.0 x 10⁶ cells/mL). The cell concentration was determined by counting the number of living cells and the number of dead cells by the trypan blue staining method.

### [Fluorescence-Activated Cell Sorting (FACS) Analysis]

The induction of the differentiation of M cells by various fatty acids (10-hydroxydecanoic acid, (+/-)-3-hydroxydecanoic acid, (9R)-9,10-dihydroxydecanoic acid, and 10-hydroxy-2-(E)-decenoic acid) was analyzed by FACS using GP2 as an indicator. As the 10-hydroxydecanoic acid (10HDAA), one produced by SIGMA-ALDRICH was used; as the (+/-)-3-hydroxydecanoic acid (3HDAA), one produced by SIGMA-ALDRICH was used; and as the 10-hydroxy-2-(E)-decenoic acid (10-HDA), one produced by Wako Pure Chemical Industries, Ltd. was used. The (9R)-9,10-dihydroxydecanoic acid (DDA-11) used was synthesized based on the method described in Tetrahedron: Asymmetry, 2009, 20, pp. 457-460, by tosylating (4R)-4-(2-hydroxyethyl)-2,2-dimethyl-1,3-dioxolane, and extending the chain with 5-bromo-1-pentene by a Grignard reaction, followed by ozone degradation, Wittig reaction with diethylphosphonic acid ethyl ester, hydrogenation by a catalytic hydrogenation reaction, removal of acetonide by acetic acid treatment, and removal of the ethyl group by potassium hydroxide treatment.

Each of the above fatty acids was prepared as a DMSO solution (10 mM). After the Caco-2 cells were seeded in a 6-well plate and allowed to form a single-layer film, each of the above fatty acids was added so that the final concentration was 100 µm. The same amount of DMSO was added as a control. The medium was as described above. After addition, the cells were cultured at 37°C in the presence of 5% CO₂ for 3 days. After culture, the cells were collected with 1 mM EDTA·2Na/PBS(-) (pH 7.2), and stained with rabbit anti-GP2 antibody (produced by IMGENEX) and Alexa 488-labeled anti-rabbit antibody (produced by Molecular probes). The stained cells were analyzed by FACS (produced by BECKMAN COULTER), and GP2 expression levels were quantified.

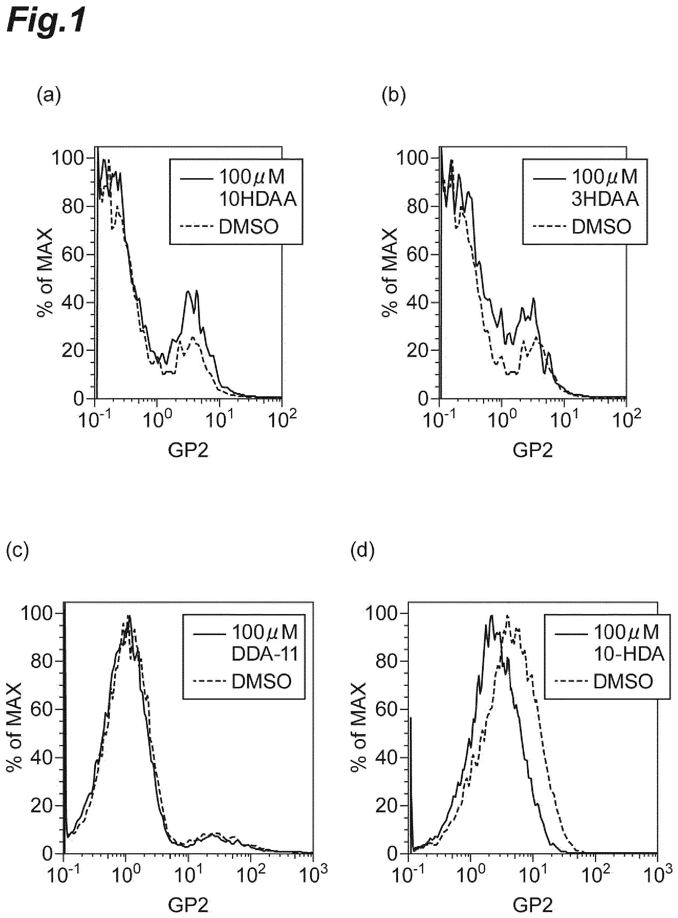

Histograms of FACS are shown in Fig. 1. As shown in Fig. 1, 10-hydroxydecanoic acid (Fig. 1(a)) and (+/-)-3-hydroxydecanoic acid (Fig. 1(b)) induced the expression of GP2. 10-hydroxydecanoic acid induced more intensely the expression of GP2. In contrast, (9R)-9,10-dihydroxydecanoic acid (Fig. 1(c)) and 10-hydroxy-2-(E)-decenoic acid (Fig. 1(d)) did not induce the expression of GP2.

### [Morphological Analysis]

After the Caco-2 cells were seeded at 2 x 10⁵ cells in a Transwell insert (6.5 mm Transwell, produced by Corning) and allowed to form a single-layer film, 10-hydroxydecanoic acid (final concentration: 100 µm) was applied from the apical side of the single-layer film, and the cells were cultured at 37°C in the presence of 5% CO₂ for 3 days. After culture, the single-layer film together with the membrane of the Transwell insert was cut, and the cell morphology was observed with an electron microscope (produced by JEOL).

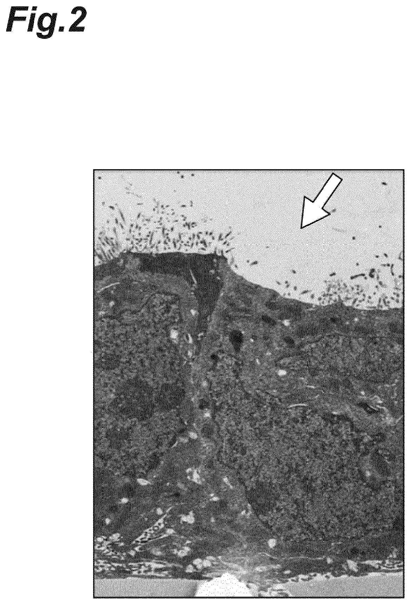

An electron micrograph is shown in Fig. 2. M cells generally have the morphological characteristics that microvilli on the apical side are shorter than those of epithelial cells in the vicinity thereof. As indicated by an arrow in Fig. 2, cells with short microvilli were observed due to the application of 10-hydroxydecanoic acid. From the results, it is considered to suggest that the Caco-2 cells were morphologically changed to M cell-like cells by the action of 10-hydroxydecanoic acid.

### [Test Example 2: M-Cell Differentiation Induction Test - in vivo Test]

### [Test Method]

A DMSO solution (10 mM) of 10-hydroxydecanoic acid was diluted 100 times with PBS(-) to prepare a test solution. Three male cynomolgus monkeys (Macaca fascicularis) (age at the start of test: 3 to 4 years old, body weight at the start of test: 3.53 to 4.36 kg) were used as test animals. About 0.1 mL of the above test solution was sprayed to each of the left and right nasal cavities of each cynomolgus monkey using a fine atomizer (Neizaru) (product number: FAN020, produced and sold by Yoshikawa Kasei Co., Ltd.). The same operation was performed using PBS(-) as a control. After this operation was repeated once a day for 3 days, the cynomolgus monkeys were dissected on day 4, and the nasopharyngeal mucosa in the pharyngeal tonsil site was collected. The collected nasopharyngeal mucosa was subjected to fluorescent immunohistostaining with Alexa 555-labeled anti-GP2 antibody (produced by IMGENEX) or Alexa 555-labeled anti-GP2 antibody (produced by Abcam), and fluorescence images were observed with an all-in-one fluorescence microscope BZ-9000 (produced by Keyence Corporation). DAPI staining was used in combination for the observation of the fluorescence images.

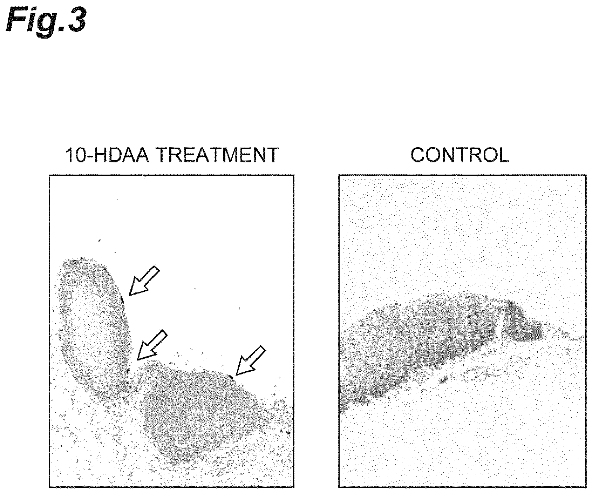

Fluorescent immunohistostaining photographs are shown in Fig. 3. In order to enhance the visibility of Fig. 3, light and darkness are reversed. As shown in Fig. 3, because the inside of the nasal cavities was exposed to 10-hydroxydecanoic acid for 3 days, GP2-positive cells (black portions shown by arrows in Fig. 3) were observed on the nasopharyngeal mucosa. In contrast, such an increase in GP2-positive cells was not observed in the control.

### [Test Example 3: Immunostimulation Test - in vivo Test]

### [Test Method] (Preparation of Capsules)

Only 2.4 mg of 10-hydroxydecanoic acid was placed in an enteric capsule to prepare a capsule containing 10-hydroxydecanoic acid (capsule A). In addition to 2.4 mg of 10-hydroxydecanoic acid, Fetuin (3 mg), an inactivated poliovirus antigen (10⁶ TCID₅₀), and an inactivated influenza virus antigen (220_HA Units) were placed as antigens in an enteric capsule to prepare a capsule containing 10-hydroxydecanoic acid and the above 3 antigens (capsule B). Further, Fetuin (3 mg), an inactivated poliovirus antigen (10⁶ TCID₅₀), and an inactivated influenza virus antigen (220_HA Units) were placed as antigens in an enteric capsule to prepare a capsule containing the above 3 antigens (capsule C).

### (Administration of Capsule)

Three male cynomolgus monkeys (Macaca fascicularis) (age at the start of test: 3 to 6 years old, body weight at the start of test: 3.0 to 6.0 kg) were used as test animals, and the above capsule A and capsule B were orally administered according to the schedule shown in the following Table 1 (1 capsule once a day) (hereinafter, "the test group"). As a control, only the capsule C was orally administered to three male cynomolgus monkeys (age at the start of test: 3 to 6 years old, body weight at the start of test: 3.0 to 6.0 kg) according to the schedule shown in the following Table 1 (the capsule A and capsule B were not administered) (hereinafter, "the control group"). The cynomolgus monkeys were dissected on day 22, and the intestinal mucosa was collected. The collected intestinal mucosa was subjected to fluorescent immunohistostaining with Alexa 555-labeled anti-GP2 antibody (produced by IMGENEX) or Alexa 555-labeled anti-GP2 antibody (produced by Abcam), and fluorescence images were observed with an all-in-one fluorescence microscope BZ-9000 (produced by Keyence Corporation). DAPI staining was used in combination for the observation of the fluorescence images.

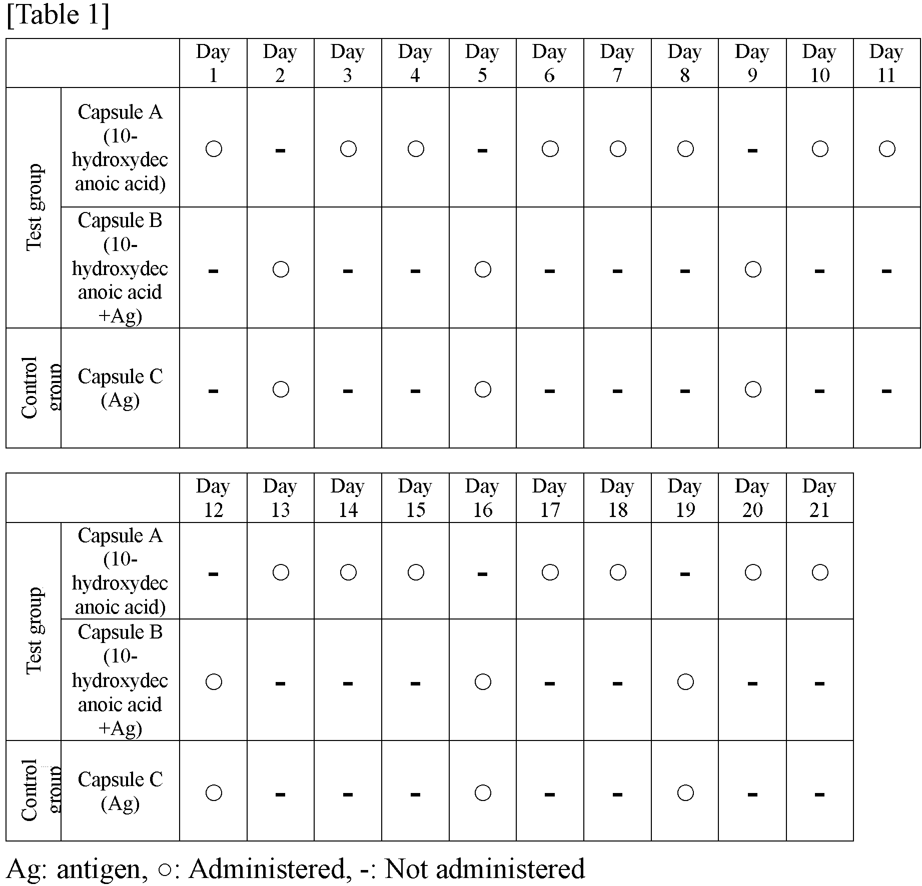

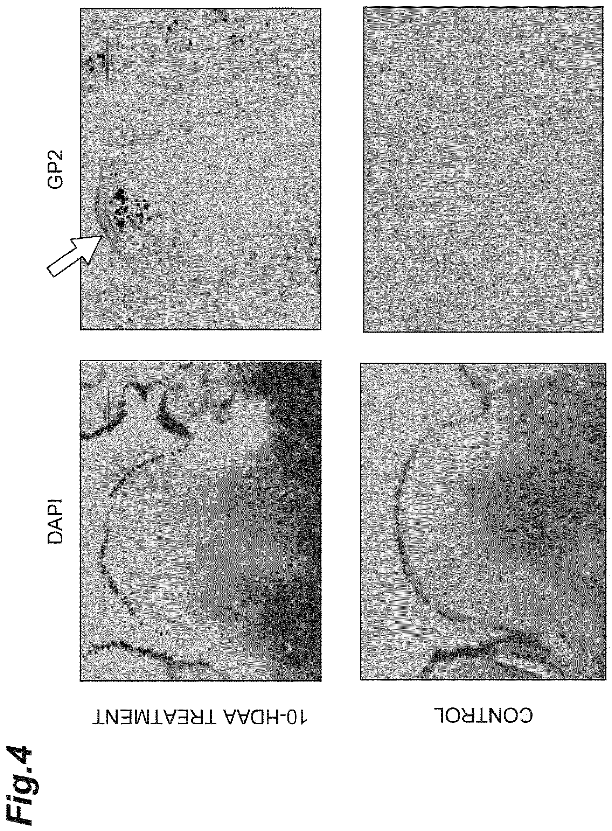

Examples of fluorescent immunohistostaining photographs are shown in Fig. 4. In order to enhance the visibility of Fig. 4, light and darkness are reversed. As shown in Fig. 4, GP2-positive cells (black portions shown by an arrow in Fig. 4) were observed on the intestinal mucosa in the test group, which was sensitized with the antigens about every 3 days while 10-hydroxydecanoic acid was continuously administered once a day. In contrast, such an increase in GP2-positive cells was not observed in the control group, which was sensitized only with the antigens about every 3 days.

### [Test Example 4: Measurement of Antibody Titers]

[Test Method] Feces of the cynomolgus monkeys raised in Test Example 3 were collected, and the anti-Fetuin IgA antibody titer, anti-poliovirus IgA antibody titer, and anti-influenza virus IgA antibody titer in the feces were measured by ELISA.

Fetuin antigen (produced by Sigma) was dissolved at 100 µg/mL in an antigen buffer (50 mmol/L Tris-HCl (pH 8.0), 10 mmol/L MgCl₂, and 0.1% Tween 80). This antigen was added to a Nunc MaxiSorp (registered trademark) flat-bottom 96-well plate to prepare an antigen plate. Influenza virus H1N1 antigen (11,000 HA Unit) and poliovirus antigen type III (0.67 x 10⁸ TCID₅₀) were diluted with 25 mL of antigen buffer, and an antigen plate was prepared in the same manner as above. For masking, a reaction was performed at 200 µl/well at 4°C overnight.

A sample buffer (0.1% sodium azide, 1 mmol/L EDTA·2Na, 0.05% Tween 20, 5% nonfat skim milk, and a PBS(-) (pH 7.2) solution of 1 mmol/L phenylmethylsulfonyl fluoride (PMSF)) was added to feces samples so that the weight ratio was 1:4. After the resultant was centrifuged at 13000 x g for 5 minutes at 4°C, the supernatant was collected to prepare a sample for ELISA. The sample for ELISA was stored in a chilling freezer. At the time of measurement, the sample for ELISA was diluted 10 times and 100 times with PBS(-).

The prepared sample was added in an amount of 50 µl to each well of the antigen plate, and shaken for 1.5 hours in a low-temperature chamber. Thereafter, washing was performed 4 times using a washing buffer (0.1% Tween 80 in MilliQ: 200 µl/well).

As a second antibody, BSA was dissolved in a second antibody buffer (0.5 M Tris-HCl, 1.75% NaCl) to 1%, and the following antibodies (a) and (b) were diluted 5000 times with this buffer.
(a) Peroxidase-Labeled Affinity Purified antibody to Monkey IgA (alpha) (Cat. No. 074-11-011, KPL)
(b) Goat Anti-monkey IgA (alpha-chain specific)-peroxidase (Affinity purified) (Cat. No. 70041, Alpha diagnostic international)

The second antibody solution was added in an amount of 50 µl to each well, and reacted for 0.75 hours. After the reaction, washing was performed 4 times using a washing buffer (0.1% Tween 80 in MilliQ: 200 µl/well).

A substrate solution (2.68 mg of TMBZ, 1.05 µl of 35% hydrogen peroxide solution, and 7 mL of EDTA·2Na (2 mM)) was added in an amount of 50 µl to each well. Finally, 50 µl of 0.3N H₂SO₄ was added to each well to terminate the reaction. Thereafter, the absorbance (450 nm/630 nm) was measured.

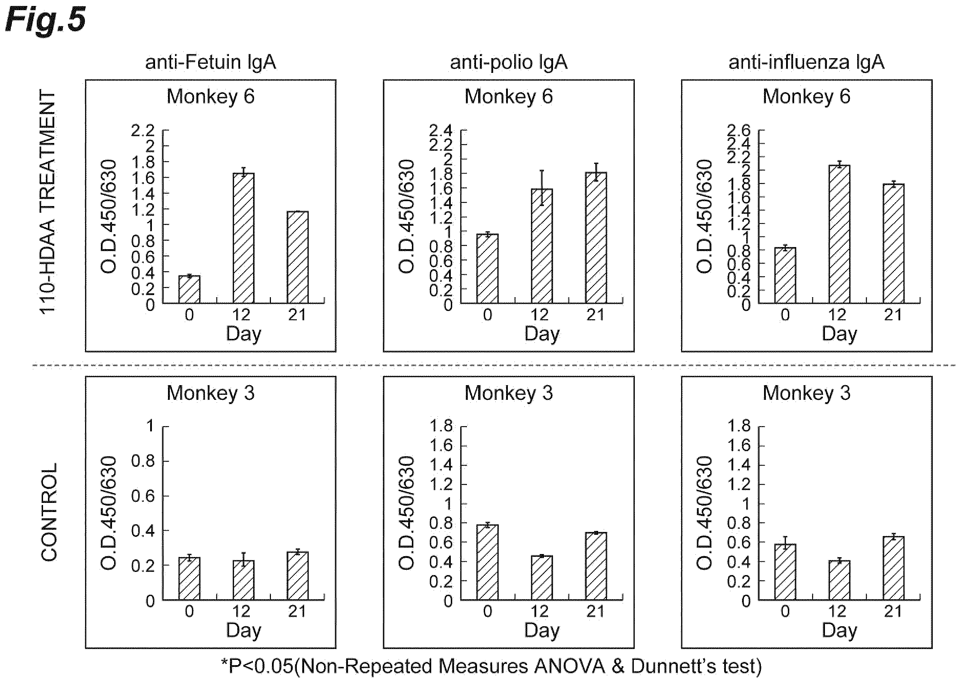

The anti-Fetuin IgA antibody titer, anti-poliovirus IgA antibody titer, and anti-influenza virus IgA antibody titer in the feces are shown in Fig. 5. As shown in Fig. 5, increases in the anti-Fetuin IgA antibody titer, the anti-poliovirus IgA antibody titer, and the anti-influenza virus IgA antibody titer were observed in the test group, which was sensitized with the antigens about every 3 days while 10-hydroxydecanoic acid was continuously administered once a day. In contrast, such increases in the antibody titers were not observed in the control group, which was sensitized only with the antigens about every 3 days. These results are considered to suggest that immune responses are induced by the continuous administration of 10-hydroxydecanoic acid. Moreover, it was indicated that the induction of immune responses by 10-hydroxydecanoic acid did not depend on the type of antigen, and was also observed in the case of inactivated antigens. Furthermore, it was indicated that the induction of immune responses was possible even in the case of simultaneous sensitization with a plurality of antigens.

## Claims

1. An agent comprising, as an active ingredient, at least one saturated fatty acid selected from the group consisting of 3-hydroxydecanoic acid and 10-hydroxydecanoic acid and salts thereof for use in mucosal immunostimulation.

2. The agent for use according to claim 1, wherein the saturated fatty acid is 10-hydroxydecanoic acid.

3. The agent for use according to claim 1 or 2, wherein the mucosal immunostimulation is based on induction of differentiation of M cells.

4. The agent for use according to any one of claims 1 to 3. wherein the agent is in the form of a food composition.

5. The agent for use according to any one of claims 1 to 3. wherein the agent is an adjuvant.

6. A vaccine preparation comprising an immunogen and at least one agent selected from the group consisting of 3-hydroxydecanoic acid and 10-hydroxydecanoic acid and salts thereof, for use as a vaccine, whereby the use includes mucosal immunostimulation.

## Patentansprüche

1. Mittel, umfassend als einen Wirkstoff mindestens eine gesättigte Fettsäure, ausgewählt aus der Gruppe bestehend aus 3-Hydroxydecansäure und 10-Hydroxydecansäure und Salzen davon, zur Verwendung bei der mukosalen Immunstimulation.

2. Mittel zur Verwendung nach Anspruch 1, wobei die gesättigte Fettsäure 10-Hydroxydecansäure ist.

3. Mittel zur Verwendung nach Anspruch 1 oder 2, wobei die mukosale Immunstimulation auf der Induktion der Differenzierung von M-Zellen beruht.

4. Mittel zur Verwendung nach einem der Ansprüche 1 bis 3, wobei das Mittel in Form einer Lebensmittelzusammensetzung vorliegt.

5. Mittel zur Verwendung nach einem der Ansprüche 1 bis 3, wobei das Mittel ein Adjuvans ist.

6. Impfstoffpräparat, umfassend ein Immunogen und mindestens ein Mittel, ausgewählt aus der Gruppe bestehend aus 3-Hydroxydecansäure und 10-Hydroxydecansäure und Salzen davon, zur Verwendung als Impfstoff, wobei die Verwendung eine mukosale Immunstimulation einschließt.

## Revendications

1. Agent comprenant, comme principe actif, au moins un acide gras saturé sélectionné dans le groupe constitué par l'acide 3-hydroxydécanoïque et l'acide 10-hydroxydécanoïque, ainsi que leurs sels, destiné à être utilisé pour l'immunostimulation des muqueuses.

2. Agent destiné à être utilisé selon la revendication 1, dans lequel l'acide gras saturé est l'acide 10-hydroxydécanoïque.

3. Agent destiné à être utilisé selon la revendication 1 ou 2, dans lequel l'immunostimulation des muqueuses est basée sur l'induction de la différenciation des cellules M.

4. Agent destiné à être utilisé selon l'une quelconque des revendications 1 à 3, dans lequel l'agent se présente sous la forme d'une composition alimentaire.

5. Agent destiné à être utilisé selon l'une quelconque des revendications 1 à 3, dans lequel l'agent est un adjuvant.

6. Préparation vaccinale comprenant une substance immunogène et au moins un agent sélectionné dans le groupe constitué par l'acide 3-hydroxydécanoïque et l'acide 10-hydroxydécanoïque, ainsi que leurs sels, destinée à être utilisée comme vaccin, dans laquelle l'utilisation inclut l'immunostimulation des muqueuses.
